(19) Europäisches Patentamt
European Patent Office
Office européen des brevets

(11) **EP 4 163 384 B1**

(12) **EUROPEAN PATENT SPECIFICATION**

(45) Date of publication and mention
of the grant of the patent:
**25.02.2026  Bulletin 2026/09**

(21) Application number: **20938627.5**

(22) Date of filing: **04.06.2020**

(51) International Patent Classification (IPC):
**G16H 50/20** (2018.01)      *G16B 20/00* (2019.01)
*C12Q 1/00* (2006.01)

(52) Cooperative Patent Classification (CPC):
**G16H 50/20;** G01N 2800/368; G16B 20/00

(86) International application number:
**PCT/CN2020/094394**

(87) International publication number:
**WO 2021/243650 (09.12.2021 Gazette 2021/49)**

(54) **METHOD FOR DETERMINING PREGNANCY STATUS OF PREGNANT WOMAN**

**VERFAHREN ZUR BESTIMMUNG DES SCHWANGERSCHAFTSZUSTANDS SCHWANGERE**

**PROCÉDÉ POUR DÉTERMINER L'ÉTAT DE GROSSESSE D'UNE FEMME ENCEINTE**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**12.04.2023  Bulletin 2023/15**

(73) Proprietor: **BGI Genomics Co., Limited
Guangdong 518083 (CN)**

(72) Inventors:
• **CHEN, Ruoyan
Shenzhen, Guangdong 518083 (CN)**
• **LIU, Siyang
Shenzhen, Guangdong 518083 (CN)**
• **JIN, Xin
Shenzhen, Guangdong 518083 (CN)**

(74) Representative: **Dehns Germany Partnerschaft
mbB
Theresienstraße 6-8
80333 München (DE)**

(56) References cited:
CN-A- 107 133 491      CN-A- 107 133 495
CN-A- 108 315 393      CN-A- 110 964 800
US-A1- 2005 074 746    US-B2- 7 541 182

• **LIN JASON ET AL: "Predicting Premature Birth
Risk with cf RNA Recommended Citation
Predicting Premature Birth Risk with cfRNA",**
SMU DATA SCIENCE REVIEW, vol. 2, no. 2, 1
January 2019 (2019-01-01), XP055820616

## Description

### TECHNICAL FIELD

The present disclosure relates to the field of biotechnology, in particular non-invasive prenatal genetic testing, and specifically to a method and apparatus for determining the pregnancy status of a pregnant woman and a corresponding method and apparatus for constructing a machine learning prediction model.

### BACKGROUND

The cell-free DNAs (cfDNA) of plasma of pregnant women contain fetal cfDNAs. These fetal cfDNAs are mainly derived from placenta, and partially derived from hemopoietic stem cells or directly derived from exchange between fetus and mother body. Studies have confirmed that the concentration of fetal cfDNAs in the plasma of pregnant women is correlated with various pregnancy complications such as premature delivery, intrauterine growth retardation, and pregnancy eclampsia.

Research articles about the correlation between fetal cfDNA concentration in the plasma of pregnant women and premature delivery have emerged constantly in recent years. However, there is no definite conclusion on the correlation between fetal cfDNA concentration and premature delivery, and there are contradictory conclusions in different research literatures.

Currently, methods for effectively predicting premature delivery based on the fetal cfDNA concentration remain to be developed. LIN JASON et al., "Predicting Premature Birth Risk with cf RNA Recommended Citation Predicting Premature Birth Risk with cfRNA″ provides a method for constructing a prediction model by using cell-free RNA data from non-invasive blood tests in conjunction with machine learning. US 7,541,182 B2 provides methods for identifying patients at risk of developing preeclampsia.

### SUMMARY

The present disclosure is provided based on the discovery and recognition by the inventors of the following facts and issues:

To date, most of clinical predictions of threatened premature delivery are conducted by detecting the secretion of Fetal Fibronectin in the vagina of pregnant women, but this method is only an auxiliary means and cannot be used as the final diagnosis basis. At present, there is no effective method to diagnose premature delivery in clinic.

Several reports have shown that the concentration of fetal cfDNAs in the plasma of pregnant women is correlated with various pregnancy complications, such as premature delivery and preeclampsia. Studies have attempted to predict premature delivery using the fetal cfDNA concentration as a marker, but eventually failed due to insufficient correlation. To date, there is no effective method to predict premature delivery using a fetal cfDNA concentration.

There is a high false-positive problem in the method for the diagnosis of premature delivery assisted with fetal fibronectin molecule in clinic. Statistics show that in pregnant women diagnosed as positive by fetal fibronectin molecule, only less than 3% of the samples were finally diagnosed as premature delivery. The high false-positive problem makes this diagnostic method questionable.

A previously reported method for predicting the premature delivery by only using a single factor, a concentration of fetal cfDNAs in the plasma of pregnant women, has the problem of insufficient correlation, failing to successfully establish an effective prediction model.

Additional aspects and advantages of the present disclosure will be set forth in part in the description which follows and, in part, will be apparent from the description, or may be learned by practice of the present disclosure.

According to one aspect of the present disclosure, provided is a method for constructing a prediction model for determining a pregnancy status of a pregnant woman according to embodiments of the present disclosure, including: (i) constructing a training set and a selective validation set, each of the training set and the validation set being composed of a plurality of pregnant woman samples each having a known pregnancy status; (ii) determining predetermined parameters of each pregnant woman sample in the training set, the predetermined parameters including a concentration of fetal cell-free nucleic acids in peripheral blood of the pregnant woman and a gestational age in week at which sampling for the peripheral blood of the pregnant woman is conducted; and (iii) constructing the prediction model based on the known pregnancy status and the predetermined parameters; wherein the gestational age in week at which the sampling is conducted is 13 to 25 weeks. According to the method provided by the embodiments of the present disclosure, a prediction model for the pregnancy status of the pregnant woman is constructed by utilizing the concentration of fetal cell-free nucleic acids obtained via one-time blood sampling for a plurality of pregnant woman samples, the gestational age in week at which the sampling is conducted, the physical signs (such as height, body weight, body mass index, and age) of the pregnant woman when the sampling is conducted, and the pregnancy status (such as premature delivery and gestational age in week at delivery) of the pregnant woman when the sampling is conducted, and the method includes two key factors, the concentration of fetal cell-free nucleic acids and the gestational age in week at which the sampling is conducted, so that the accuracy of the model is improved.

According to embodiments of the present disclosure, the above-mentioned method may further have at least one of the following additional technical features:

According to embodiments of the present disclosure, the pregnancy status includes a delivery interval of the pregnant woman. The method according to the embodiments of the present disclosure can be used to predict the probability of premature delivery, intrauterine growth retardation of a fetus at the gestational age in week at delivery, and other pregnancy complications associated with the concentration of fetal cell-free nucleic acids.

The inventors found that there was a weak correlation between fetal concentration and premature delivery when the gestational age in week at which the blood sampling is conducted is 12 weeks or less or between 26 weeks and 30 weeks, while there was a strong correlation between fetal concentration and premature delivery when the gestational age in week at which the blood sampling is conducted is 13 to 25 weeks.

According to embodiments of the present disclosure, the prediction model is at least one of a linear regression model, a logistic regression model, or a random forest. According to the method of embodiments of the present disclosure, the prediction model may be theoretically any statistical model that generalizes different difference distributions.

According to embodiments of the present disclosure, the predetermined parameters further include a height, a body weight, and an age of the pregnant woman.

According to embodiments of the present disclosure, the step (iii) includes determining, by using the training set and the validation set, numerical values of $\beta_0$, $\beta_{icff}$, $\beta_{isample}$, $\beta_{iheight}$, $\beta_{iweigh}$, $\beta_{iage}$, and $\varepsilon_i$ for the following formula: $l_i = \beta_0 + \beta_{icff}x_{icff} + \beta_{isample}x_{isample} + \beta_{iheight}x_{iheight} + \beta_{iweight}x_{iweight} + \beta_{iage}x_{iage} + \varepsilon_i$, where $i = 1, ..., p$, wherein $i$ represents a serial number of a pregnant woman sample in the training set; $l_i$ is a value determined for the known pregnancy status of the pregnant woman sample No.$i$, wherein $l_i$ is 1 for the pregnant woman sample with premature delivery and $l_i$ is 0 for the pregnant woman sample with full-term delivery; $x_{icff}$ represents the concentration of fetal cell-free nucleic acids of the pregnant woman sample No.$i$; $x_{isample}$ represents the gestational age in week at which the sampling for the peripheral blood of the pregnant woman sample No.$i$ is conducted; $x_{iheight}$ represents the height of the pregnant woman sample No. $i$; $x_{iweight}$ represents the body weight for the pregnant woman sample No.$i$; $x_{iage}$ represents the age of the pregnant woman sample No.$i$, and $\varepsilon_i$ represents a sequencing error of the peripheral blood of the pregnant woman sample No.$i$.

In a second aspect of the present disclosure, provided is a system for constructing a prediction model for determining a pregnancy status of a pregnant woman according to embodiments of the present disclosure, including: a training set construction module configured to construct a training set composed of a plurality of pregnant woman samples each having a known pregnancy status; a predetermined parameter determination module connected to the training set construction module and configured to determine predetermined parameters of each pregnant woman sample in the training set, the predetermined parameters including a concentration of fetal cell-free nucleic acids in peripheral blood of the pregnant woman and a gestational age in week at which sampling for the peripheral blood of the pregnant woman is conducted; and a prediction model construction module connected to the predetermined parameter determination module and configured to construct the prediction model based on the known pregnancy status and the predetermined parameters. According to the embodiments of the present disclosure, the system constructs a prediction model for a pregnancy status of a pregnant woman based on the concentration of fetal cell-free DNA obtained via one-time blood sampling for a plurality of pregnant woman samples, the gestational age in week at which the sampling is conducted, the physical signs (such as height, body weight, body mass index, and age) of the pregnant woman when the sampling is conducted, and the pregnancy status (such as premature delivery and gestational age in week at delivery ) of the pregnant woman when the sampling is conducted, and the apparatus uses two key factors, the concentration of fetal cell-free DNA and the gestational age in week at which the sampling is conducted, as the key parameters for constructing the model, so that the accuracy of the constructed model is improved.

According to an embodiment of the present disclosure, the above-mentioned method may further have at least one of the following additional technical features:

According to an embodiment of the present disclosure, the pregnancy status includes a delivery interval of the pregnant woman. The system according to the embodiments of the present disclosure can be used to predict the probability of premature delivery, intrauterine growth retardation of a fetus at the gestational age in week at delivery, and other pregnancy complications associated with the concentration of fetal cell-free nucleic acids.

According to embodiments of the present disclosure, the gestational age in week at which sampling is conducted is 13 to 25 weeks. The inventors found that there was a weak correlation between fetal concentration and premature delivery when the gestational age in week at which the blood sampling is conducted is 12 weeks or less or between 26 weeks and 30 weeks, while there was a strong correlation between fetal concentration and premature delivery when the gestational age in week at which the blood sampling is conducted is 13 to 25 weeks.

According to embodiments of the present disclosure, the prediction model may be theoretically any statistical model that generalizes different difference distributions. According to a specific embodiment of the present disclosure, the prediction model is at least one of a linear regression model, a logistic regression model, or a random forest.

According to embodiments of the present disclosure, the predetermined parameters further include a height, a body weight, and an age of the pregnant woman.

According to embodiments of the present disclosure, the prediction model construction module is configured to determine, by using the training set and a validation set, numerical values of $\beta_0$, $\beta_{icff}$, $\beta_{isample}$, $\beta_{iheight}$, $\beta_{iweight}$, $\beta_{iage}$, and $\varepsilon_i$ for the following formula: $l_i = \beta_0 + \beta_{icff}x_{icff} + \beta_{isample}x_{isample} + \beta_{iheight}x_{iheight} + \beta_{iweight}x_{iweight} + \beta_{iage}x_{iage} + \varepsilon_i$, where $i = 1, ..., p$, wherein $i$ represents a serial number of the pregnant woman sample in the training set; $l_i$ is a value determined for the known pregnancy status of the pregnant woman sample No.$i$, $l_i$ is 1 for the pregnant woman sample with premature delivery, and $l_i$ is 0 for the pregnant woman sample with full-term delivery; $x_{icff}$ represents the concentration of fetal cell-free nucleic acids of the pregnant woman sample No.i; $x_{isample}$ represents the gestational age in week at which the sampling for the peripheral blood of the pregnant woman sample No.$i$ is conducted; $x_{iheight}$ represents the height of the pregnant woman sample No.$i$; $x_{iweight}$ represents the body weight of the pregnant woman sample No.$i$; $x_{iage}$ represents the age of the pregnant woman sample No.$i$; and $\varepsilon_i$ represents a sequencing error of the peripheral blood of the pregnant woman sample No.i.

In a third aspect of the present disclosure, provided is a method for determining a pregnancy status of a pregnant woman. According to embodiments of the present disclosure, the method includes: (1) determining predetermined parameters of the pregnant woman, the predetermined parameters including a concentration of fetal cell-free nucleic acids in peripheral blood of the pregnant woman and a gestational age in week at which sampling for the peripheral blood of the pregnant woman is conducted; and (2) determining the pregnancy status of the pregnant woman based on the predetermined parameters and the prediction model constructed according to the method for constructing the prediction model. The method according to the embodiments of the present disclosure can quickly and accurately predict the pregnancy status of the pregnant woman based on information about the concentration of fetal cell-free nucleic acids in the peripheral blood of the pregnant woman obtained via one-time blood sampling at early pregnancy, the gestational age in week at which the sampling for the peripheral blood is conducted, and the physical sign data of the pregnant woman, the pregnancy status including the gestational age in week at delivery, the probability of premature delivery, the intrauterine growth retardation of the fetus, and other pregnancy complications associated with the concentration of fetal cell-free nucleic acids.

According to an embodiment of the present disclosure, the above-mentioned method may further have at least one of the following additional technical features:

According to embodiments of the present disclosure, the pregnancy status includes a delivery interval of the pregnant woman. The delivery interval refers to the gestational age in week at delivery. The method according to the embodiments of the present disclosure can effectively predict the gestational age in week at delivery and the probability of premature delivery of a pregnant woman. In addition, the method according to the embodiments of the present disclosure can also effectively predict pregnancy complications associated with the concentration of fetal cell-free nucleic acids, such as the probability of premature delivery and intrauterine growth retardation of a fetus at the gestational age in week at delivery.

According to embodiments of the present disclosure, the gestational age in week at which the sampling is conducted is 13 to 25 weeks. The inventors found that there was a weak correlation between fetal concentration and premature delivery when the gestational age in week at which the blood sampling is conducted is 12 weeks or less or between 26 weeks and 30 weeks, while there was a strong correlation between fetal concentration and premature delivery when the gestational age in week at which the blood sampling is conducted is 13 to 25 weeks.

According to embodiments of the present disclosure, the prediction model may be theoretically any statistical model that generalizes different difference distributions. According to a specific embodiment of the present disclosure, the predetermined prediction model is at least one of a linear regression model, a logistic regression model, or a random forest.

According to embodiments of the present disclosure, the predetermined parameters further include a height, a body weight, and/or an age of the pregnant woman, and the prediction model is adapted to calculate the delivery interval of the pregnant woman based on the following formula: $l = \beta_0 + \beta_{cff}x_{cff} + \beta_{sample}x_{sample} + \beta_{height}x_{height} + \beta_{weight}x_{weight} + \beta_{age}x_{age} + \varepsilon$, wherein $l$ is a parameter determined based on a probability of premature delivery of the pregnant woman; $\beta_0$, $\beta_{cff}$, $\beta_{sample}$, $\beta_{height}$, $\beta_{weight}$, and $\varepsilon$ are each independently a predetermined coefficient; $x_{cff}$ is the concentration of fetal cell-free nucleic acids of the pregnant woman; $x_{sample}$ is the gestational age in week at which the sampling for the maternal peripheral blood of the pregnant woman is conducted; $x_{height}$ is the height of the pregnant woman; $x_{weight}$ is the body weight of the pregnant woman; $x_{age}$ is the age of the pregnant woman, and $\varepsilon_i$ is a sequencing error of a peripheral blood sample of the pregnant woman. According to the embodiments of the present disclosure, the coefficients $\beta_0$, $\beta_{cff}$, $\beta_{sample}$, $\beta_{height}$, and $\beta_{weight}$ may be obtained based on a predetermined training set, one or several of which may be selected, and the pregnant woman's body mass index (BMI) may be added as one of the coefficients.

According to embodiments of the present disclosure, $l$ is determined based on the following formula:

$$l = log_b \frac{P}{1-p}$$

, where b is a base number of log and is generally a constant e, and p is the probability of premature delivery of the pregnant woman.

In a fourth aspect of the present disclosure, provided is an apparatus for determining a pregnancy status of a pregnant woman. According to embodiments of the present disclosure, the apparatus includes: a parameter determination module configured to determine predetermined parameters of the pregnant woman, the predetermined parameters including a concentration of fetal cell-free nucleic acids in peripheral blood of the pregnant woman and a gestational age in week at which sampling for the peripheral blood of the pregnant woman is conducted; and a pregnancy status determination module connected to the parameter determination module and configured to determine the pregnancy status of the pregnant woman based on the predetermined parameters and the prediction model. The apparatus according to the embodiments of the present disclosure can quickly and accurately predict the pregnancy status of the pregnant woman based on the information about the concentration of fetal cell-free nucleic acids obtained via one-time blood sampling at early pregnancy of the pregnant woman, the gestational age in week at which the sampling for the peripheral blood is conducted, and the physical sign data of the pregnant woman, the pregnancy status including the gestational age in week at delivery, the probability of premature delivery, the intrauterine growth retardation of the fetus and other pregnancy complications associated with the concentration of fetal cell-free nucleic acids.

According to embodiments of the present disclosure, the above-mentioned apparatus may further have the following additional technical features:

According to embodiments of the present disclosure, the pregnancy status includes a delivery interval of the pregnant woman. The method according to the embodiments of the present disclosure can predict the probability of premature delivery, intrauterine growth retardation of the fetus at the gestational age in week at delivery, and other pregnancy complications associated with the concentration of fetal cell-free nucleic acids.

According to embodiments of the present disclosure, the gestational age in week at which the sampling is conducted is 13 to 25 weeks. The inventors found that there was a weak correlation between fetal concentration and premature delivery when the gestational age in week at which the blood sampling is conducted is 12 weeks or less or between 26 weeks and 30 weeks, while the gestational age in week at which the blood sampling is conducted is 13 to 25 weeks.

According to embodiments of the present disclosure, the predetermined prediction model is at least one of a linear regression model, a logistic regression model, or a random forest. According to a specific embodiment of the present disclosure, the prediction model may be theoretically any statistical model that generalizes different difference distributions.

According to embodiments of the present disclosure, the predetermined parameters further include a height, a body weight, and an age of the pregnant woman, and the prediction model is adapted to calculate a delivery interval of the pregnant woman based on the following formula: $l = \beta_0 + \beta_{cff}x_{cff} + \beta_{sample}x_{sample} + \beta_{height}x_{height} + \beta_{weight}x_{weight} + \beta_{age}x_{age} + \varepsilon$, wherein $l$ is a parameter determined based on the probability of premature delivery of the pregnant woman; $\beta_0$, $\beta_{cff}$, $\beta_{sample}$, $\beta_{height}$, $\beta_{weight}$, and $\varepsilon$ are each independently a predetermined coefficient; $x_{cff}$ is the concentration of fetal cell-free nucleic acids of the pregnant woman; $x_{sample}$ is the gestational age in week at which the sampling for the peripheral blood of the pregnant woman is conducted; $x_{height}$ is the height of the pregnant woman; $x_{weight}$ is the body weight of the pregnant woman; $x_{age}$ is the age of the pregnant woman, and $\varepsilon$ is a sequencing error of a peripheral blood sample of the pregnant woman. According to embodiments of the present disclosure, the coefficients $\beta_0$, $\beta_{cff}$, $\beta_{sample}$, $\beta_{height}$, and $\beta_{weight}$ may be freely selected as needed, for example, the pregnant woman BMI may be additionally added as one of the coefficients.

According to embodiments of the present disclosure, $l$ is determined based on the following formula:

$$l = log_b \frac{P}{1-p}$$

, wherein b is a base number of log and is generally a constant e, and p is the probability of premature delivery of the pregnant woman.

In a fifth aspect of the present disclosure, provided is a computer-readable storage medium having a computer program stored thereon. The program, when executed by a processor, implements the steps of the above-described method for constructing the prediction model. Thus, the above-described method for constructing the prediction model can be effectively implemented, so that the prediction model can be effectively constructed, and the prediction model can be then used to perform prediction on an unknown sample to determine the pregnancy status of the pregnant woman to be detected.

**BRIEF DESCRIPTION OF DRAWINGS**

The foregoing and/or additional aspects and advantages of the present disclosure will become apparent and readily appreciated from the following description of the embodiments, taken in conjunction with the accompanying drawings, in which:

FIG. 1 is a graph showing the correlation of premature delivery and fetal cfDNA concentrations in different gestational ages in week at which blood sampling was conducted according to an embodiment of the present disclosure;

FIG. 2 is a graph showing changes in specificity, sensitivity, and accuracy under different premature delivery probability thresholds that were set when predicting premature delivery using a test data set according to an embodiment of the present disclosure;

FIG. 3 is a graph showing the distribution of predicted gestational ages in week at delivery and actual gestational ages in week at delivery according to an embodiment of the present disclosure;

FIG. 4 is a schematic flowchart of a method for constructing a prediction model according to an embodiment of the present disclosure;

FIG. 5 is a block diagram of a system for constructing a prediction model according to an embodiment of the present disclosure;

FIG. 6 is a schematic flowchart of a method for determining a pregnancy status of a pregnant woman according to an embodiment of the present disclosure; and

FIG. 7 is a block diagram of an apparatus for a method for determining a pregnancy status of a pregnant woman according to an embodiment of the present disclosure.

## DESCRIPTION OF EMBODIMENTS

Embodiments of the present disclosure will be described in detail below, examples of which are illustrated in the accompanying drawings. The examples described below with reference to the accompanying drawings are illustrative, which are merely intended to explain the present disclosure, rather than to limit the present disclosure.

Explanation of Terms

As used herein, the terms "first", "second", "third", and other similar terms, unless specifically stated otherwise, are used for descriptive purposes to distinguish one from another and are not intended to imply or express any differences in order or importance, and it is not intended to mean that a content defined by terms such as "first", "second", "third" and the like consists of only one element.

In the present disclosure, unless otherwise clearly specified and limited, the terms "installation", "interconnection", "connection" and "fixation" etc. are intended to be understood in a broad sense, for example, it may be a fixed connection, removable connection or integral connection; may be a mechanical connection or electrical connection; may be a direct connection or indirect connection using an intermediate; and may be a communication within two elements or an interaction relationship between the two elements, unless explicitly limited otherwise. A person of ordinary skill in the art can understand specific meanings of these terms in the present disclosure based on specific situations.

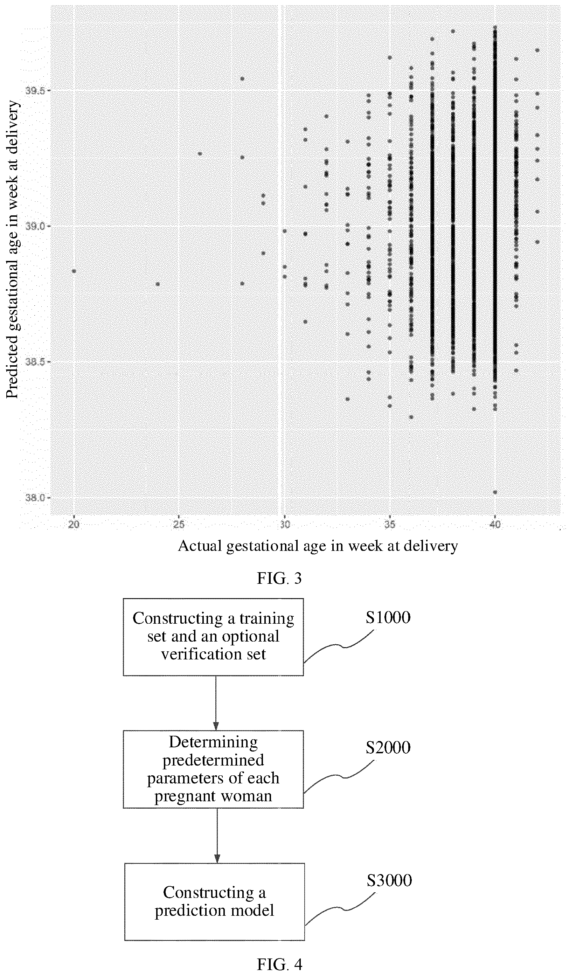

According to one aspect of the present disclosure, a method for constructing a prediction model is provided. According to an embodiment of the present disclosure, referring to FIG. 4, the prediction model is configured to determine a pregnancy status of a pregnant woman, and the method includes:

S 1000, constructing a training set and a selective validation set, each of the training set and the validation set being composed of a plurality of pregnant woman samples each having a known pregnancy status;

S2000, determining predetermined parameters of each pregnant woman sample in the training set, the predetermined parameters including a concentration of fetal cell-free nucleic acids in peripheral blood of the pregnant woman and a gestational age in week at which sampling for the peripheral blood of the pregnant woman is conducted; and

S3000, constructing the prediction model based on the known pregnancy status and the predetermined parameters. The method according to the embodiment of the present disclosure constructs a prediction model for the pregnancy status of the pregnant woman based on the concentration of fetal cell-free nucleic acids obtained via one-time blood sampling for a plurality of pregnant woman samples, the gestational age in week at which the sampling is conducted, the physical signs (such as height, body weight, BMI, and age) of the pregnant woman when the sampling is conducted, and the pregnancy status (such as premature delivery and gestational age in week at delivery) of the pregnant woman when the sampling is conducted, and the method includes two key factors, the concentration of fetal cell-free nucleic acids and the sampling gestational age in week, so that the accuracy of the model is improved. According to an embodiment of the present disclosure, the concentration of fetal cell-free nucleic acids is obtained by data processing using sequencing data of the cell-free nucleic acids in the plasma of a pregnant woman as input data, and specifically includes: after the quality control of raw sequencing data (fq format) is finished, aligning the sequencing data to human reference chromosomes by using alignment software (such as a samse mode in BWA); using sequencing data quality control software (such as Picard) to remove the repeated reads in the alignment results and calculate the repetition rate; completing the local correction of the alignment results by using mutation detection algorithm (such as Base Quality Score Recalibration BQSR function in GATK); and calculating the average depth of different chromosomes in each sample by using coverage depth calculation software (such as Depth of Coverage function in GATK). For male fetus samples, the mean depth of coverage of the unique alignment reads matching the non-homologous region of Y chromosome is calculated, and the ratio of this mean depth to the mean depth of the unique alignment reads matching autosome is the concentration of fetal cell-free nucleic acids. For female fetus samples, calculation can be performed using existing methods for calculating the concentration of fetal cell-free nucleic acids based on low-depth sequencing data of maternal plasma.

According to a specific embodiment of the present disclosure, in the method of the present disclosure, pregnant woman samples are selected as a training set and a validation set, a prediction model is constructed based on the known pregnancy status, concentration of fetal cell-free nucleic acids, height, body weight, age, BMI, and gestational age in week at which blood sampling is conducted (13 to 25 weeks) in the training set, and the magnitude of each fixed coefficient in the prediction model formula is then determined, so as to predict the pregnancy status of the pregnant woman to be detected.

According to an embodiment of the present disclosure, the pregnancy status includes a delivery interval of the pregnant woman. The method according to the embodiment of the present disclosure can be used to predict the probability of premature delivery, intrauterine growth retardation of a fetus at the gestational age in week at delivery, and other pregnancy complications associated with the concentration of fetal cell-free nucleic acids.

According to an embodiment of the present disclosure, the gestational age in week at which the sampling is conducted is 13 to 25 weeks. The inventors found that there was a weak correlation between fetal cell-free nucleic acid concentration and premature delivery when the gestational age in week at which the blood sampling is conducted is 12 weeks or less or between 26 weeks and 30 weeks, while there was a strong correlation between fetal concentration and premature delivery when the gestational age in week at which the blood sampling is conducted is 13 to 25 weeks. Generally, there is a problem of weak correlation in the prediction of the pregnancy status of pregnant women using the concentration of fetal cell-free nucleic acids. According to the method of the embodiment of the present disclosure, the gestational age in week at which sampling is conducted is added as one of the parameters for constructing the prediction model, which improves the accuracy of prediction. Different pregnant woman samples can be used as model construction samples only with one-time blood sampling within a gestational age of 13 to 25 weeks, avoiding the risk and cost of repeated blood samplings for pregnant woman samples in the process of sample collection.

According to an embodiment of the present disclosure, the prediction model is at least one of a linear regression model, a logistic regression model, or a random forest. According to an embodiment of the present disclosure, the prediction model may be theoretically any statistical model that generalizes different difference distributions.

According to an embodiment of the present disclosure, the predetermined parameters further include a height, a body weight, and an age of the pregnant woman.

According to an embodiment of the present disclosure, the step (iii) includes determining, by using the training set and the validation set, numerical values of $\beta_0$, $\beta_{icff}$, $\beta_{isample}$, $\beta_{iheight}$, $\beta_{iweight}$, $\beta_{iage}$, and $\varepsilon_i$ for the following formula: $l_i = \beta_0 + \beta_{icff}x_{icff} + \beta_{isample}x_{isample} + \beta_{iheight}x_{iheight} + \beta_{iweight}x_{iweight} + \beta_{iage}x_{iage} + \varepsilon_i$, where $i = 1, ..., p$, wherein $i$ represents a serial number of the pregnant woman sample in the training set; $l_i$ is a value determined for the known pregnancy status of the pregnant woman sample No.i, wherein $l_i$ is 1 for the pregnant woman sample with premature delivery and $l_i$ is 0 for the pregnant woman sample with full-term delivery; $x_{icff}$ represents the concentration of fetal cell-free nucleic acids of the pregnant woman sample No.$i$; $x_{isample}$ represents the gestational age in week at which the sampling for the peripheral blood of the pregnant woman sample No.$i$ is conducted; $x_{iheight}$ represents the height of the pregnant woman sample No.$i$; $x_{iweight}$ represents the body weight for the pregnant woman sample No.$i$; $x_{iage}$ represents the age of the pregnant woman sample No.$i$; and $\varepsilon_i$ represents a sequencing error of the peripheral blood of the pregnant woman sample No.$i$. It should be noted that $\varepsilon$ is the random error generated by the sequencer during the sequencing process, and this value is associated with the sequencing batch but independent of the pregnant woman sample, and will be directly generated by the sequencer when downloading the sequencing data from the sequencer.

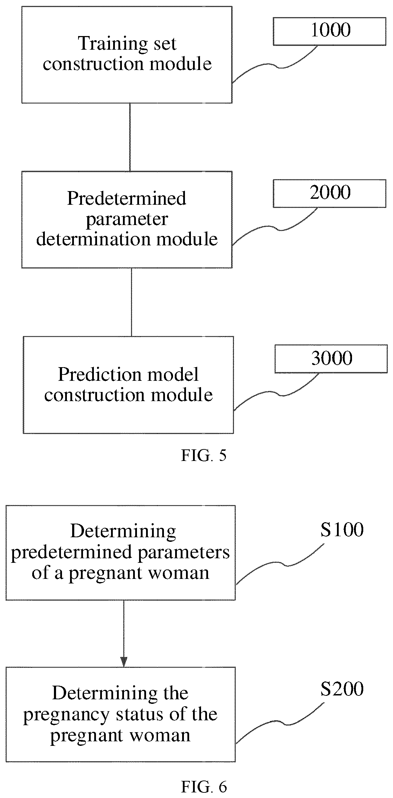

According to a second aspect of the present disclosure, a system for constructing a prediction model is provided. According to an embodiment of the present disclosure, the prediction model is used to determine a pregnancy status of a pregnant woman, and with reference to FIG. 5, the apparatus includes: a training set construction module 1000 configured to construct a training set composed of a plurality of pregnant woman samples each having a known pregnancy status; a predetermined parameter determination module 2000 connected to the training set construction module 1000 and configured to determine predetermined parameters of each pregnant woman sample in the training set, the predetermined parameters including a concentration of fetal cell-free nucleic acids in peripheral blood of the pregnant woman and a gestational age in week at which the sampling for the peripheral blood of the pregnant woman is conducted; and a prediction model construction module 3000 connected to the predetermined parameter determination module 2000 and configured to construct the prediction model based on the known pregnancy status and the predetermined parameters. The system according to the embodiment of the present disclosure constructs a prediction model for the pregnancy status of a pregnant woman based on the concentration of fetal cell-free nucleic acids obtained via one-time blood sampling for a plurality of pregnant woman samples, the gestational age in week at which the sampling is conducted, the physical signs (such as height, body weight, BMI, and age) of the pregnant woman when the sampling is conducted, and the pregnancy status (such as premature delivery and gestational age in week at delivery) of the pregnant woman when the sampling is conducted. The apparatus uses two key factors, the concentration of fetal cell-free nucleic acids and the gestational age in week at which the sampling is conducted, as the key parameters for constructing the model, so that the accuracy of the constructed model is improved.

According to an embodiment of the present disclosure, the pregnancy status includes a delivery interval of the pregnant woman. The method according to the embodiment of the present disclosure can be used to predict the probability of premature delivery, intrauterine growth retardation of a fetus at the gestational age in week at delivery, and other pregnancy complications associated with the concentration of fetal cell-free nucleic acids.

According to an embodiment of the present disclosure, the gestational age in week at which the sampling is conducted is 13 to 25 weeks. The inventors found that there was a weak correlation between fetal concentration and premature delivery when the gestational age in week at which the blood sampling is conducted is 12 weeks or less or between 26 weeks and 30 weeks, while there was a strong correlation between fetal concentration and premature delivery when the gestational age in week at which the blood sampling is conducted is 13 to 25 weeks. Generally, there is a problem of weak correlation in the prediction of the pregnancy status of pregnant women using the concentration of fetal cell-free nucleic acids. According to the system of the embodiment of the present disclosure, the gestational age in week at which the sampling is conducted is added as one of the parameters for constructing the prediction model, which improves the accuracy of prediction. Different pregnant woman samples can be used as model construction samples only with one-time blood sampling within the gestational age of 13 to 25 weeks, avoiding the risk and cost of repeated blood samplings for pregnant woman samples in the process of sample collection.

According to an embodiment of the present disclosure, the prediction model is at least one of a linear regression model, a logistic regression model, or a random forest. In the system according to an embodiment of the present disclosure, the prediction model may be theoretically any statistical model that generalizes different difference distributions.

According to an embodiment of the present disclosure, the predetermined parameters further include a height, a body weight, and an age of the pregnant woman.

According to an embodiment of the present disclosure, the prediction model construction module is configured to determine, by using the training set and a validation set, numerical values of $\beta_0$, $\beta_{icff}$, $\beta_{isample}$, $\beta_{iheight}$, $\beta_{iweight}$, $\beta_{iage}$, and $\varepsilon_i$ for the following formula: $I_i = \beta_0 + \beta_{icff}x_{icff} + \beta_{isample}x_{isample} + \beta_{iheight}x_{iheight} + \beta_{iweight}x_{iweight} + \beta_{iage}x_{iage} + \varepsilon_i$, where $i = 1, ..., p$, wherein $i$ represents a serial number of the pregnant woman sample in the training set; $I_i$ is a value determined for the known pregnancy status of the pregnant woman sample No.$i$, wherein $I_i$ is 1 for the pregnant woman sample with premature delivery and $I_i$ is 0 for the pregnant woman sample with full-term delivery; $x_{icff}$ represents the concentration of fetal cell-free nucleic acids of the pregnant woman sample No.$i$; $x_{isample}$ represents the gestational age in week at which the sampling for the peripheral blood of the pregnant woman sample No.$i$ is conducted; $x_{iheight}$ represents the height of the pregnant woman sample No.$i$; $x_{iweight}$ represents the body weight for the pregnant woman sample No.$i$; $x_{iage}$ represents the age of the pregnant woman sample No.$i$; and $\varepsilon_i$ represents a sequencing error of the peripheral blood of the pregnant woman sample No.$i$.

In a third aspect, the present disclosure provides a method for determining a pregnancy status of a pregnant woman. According to an embodiment of the present disclosure, referring to FIG. 6, the method includes:

S100, determining predetermined parameters of the pregnant woman, the predetermined parameters including a concentration of fetal cell-free nucleic acids in peripheral blood of the pregnant woman and a gestational age in week at which sampling for the peripheral blood of the pregnant woman is conducted; and

S200, determining the pregnancy status of the pregnant woman based on the predetermined parameters and the prediction model. According to the method of an embodiment of the present disclosure, the concentration of fetal cell-free nucleic acids is obtained by data processing using sequencing data of the cell-free nucleic acids in the plasma of the pregnant woman as input data, specifically including: after the quality control of raw sequencing data (fq format) is finished, aligning the sequencing data to human reference chromosomes by using alignment software (such as a samse mode in BWA); using sequencing data quality control software (such as Picard) to remove the repeated reads in the alignment results and calculate the repetition rate; completing the local correction of the alignment results by using mutation detection algorithm (such as Base Quality Score Recalibration BQSR function in GATK); and calculating the average depth of different chromosomes in each sample by using coverage depth calculation software (such as Depth of Coverage function in GATK). For male fetus samples, the mean depth of coverage of the unique alignment reads matching the non-homologous region of Y chromosome is calculated, and the ratio of this mean depth to the mean depth of the unique alignment reads matching autosome is the concentration of fetal cell-free nucleic acids. For female fetus samples, calculation can be performed using existing methods for calculating the concentration of fetal cell-free nucleic acids based on low-depth sequencing data of maternal plasma.

According to an embodiment of the present disclosure, the pregnancy status includes a delivery interval of the pregnant woman. The method according to the embodiment of the present disclosure can be used to predict the probability of premature delivery, intrauterine growth retardation of a fetus at the gestational age in week at delivery, and other pregnancy complications associated with the concentration of fetal cell-free nucleic acids.

According to an embodiment of the present disclosure, the gestational age in week at which the sampling is conducted is 13 to 25 weeks. The inventors found that there was a weak correlation between fetal concentration and premature delivery when the gestational age in week at which the blood sampling is conducted is 12 weeks or less or between 26 weeks and 30 weeks, while there was a strong correlation between fetal concentration and premature delivery when the gestational age in week at which the blood sampling is conducted is 13 to 25 weeks. Generally, there is a problem of weak correlation in the prediction of the pregnancy status of pregnant women using the concentration of fetal cell-free nucleic acids. According to the method of the embodiment of the present disclosure, the gestational age in week at which the sampling is conducted is added as one of the parameters for constructing the prediction model, which improves the accuracy of prediction, and blood sampling of the pregnant women only need to be conducted once within the gestational age of 13 to 25 weeks, which reduces the cost and risk of multiple blood samplings.

According to an embodiment of the present disclosure, the predetermined prediction model is at least one of a linear regression model, a logistic regression model, or a random forest. According to an embodiment of the present disclosure, the prediction model may be theoretically any statistical model that generalizes different difference distributions.

According to a specific embodiment of the present disclosure, the method of the present disclosure constructs a prediction model based on the known pregnancy status, concentration of fetal cell-free nucleic acids, height, body weight, age, BMI, and gestational age in week (13 to 25 weeks) at which blood sampling is conducted, and determines the magnitude of each fixed coefficient in the prediction model formula, so as to predict the pregnancy status of the pregnant woman to be detected. At the gestational age of 13 to 25 weeks, the peripheral blood of the pregnant woman to be tested is collected to detect the concentration of fetal cell-free nucleic acids, and the information about the concentration of fetal cell-free nucleic acids, height, body weight, age, BMI, and gestational age in week of the pregnant woman are input to the prediction model, so as to obtain prediction information of the pregnancy status of the pregnant woman to be tested.

According to a specific embodiment of the present disclosure, the predetermined parameters further include a height, a body weight, and an age of the pregnant woman, and the prediction model is adapted to calculate a delivery interval of the pregnant woman based on the following formula: $l = \beta_0 + \beta_{cff}x_{cff} + \beta_{sample}x_{sample} + \beta_{height}x_{height} + \beta_{weight}x_{weight} + \beta_{age}x_{age} + \varepsilon$, wherein $l$ is a parameter determined based on the probability of premature delivery of the pregnant woman; $\beta_0$, $\beta_{cff}$, $\beta_{sample}$, $\beta_{height}$, $\beta_{weight}$, and $\varepsilon$ are each independently a predetermined coefficient; $x_{cff}$ is the concentration of fetal cell-free nucleic acids of the pregnant woman; $x_{sample}$ is the gestational age in week at which the sampling for the peripheral blood of the pregnant woman is conducted; $x_{height}$ is the height of the pregnant woman; $x_{weight}$ is the body weight of the pregnant woman; $x_{age}$ is the age of the pregnant woman, and $\varepsilon_i$ is a sequencing error of a peripheral blood sample of the pregnant woman. According to the method of an embodiment of the present disclosure, the coefficients $\beta_0$, $\beta_{cff}$, $\beta_{sample}$, $\beta_{height}$, and $\beta_{weight}$ may be freely selected as needed, for example, the pregnant woman BMI may be additionally added as one of the coefficients.

According to an embodiment of the present disclosure, $l$ is determined based on the following formula:

$$l = log_b \frac{P}{1-p}$$, wherein b is a base number of log and is generally a constant e, and p is the probability of premature delivery of the pregnant woman.

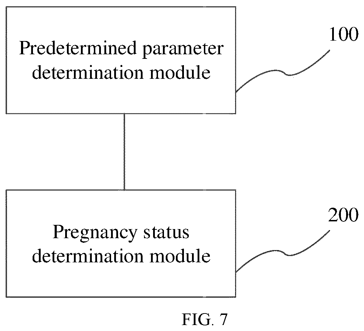

In a fourth aspect of the present disclosure, the present disclosure provides an apparatus for determining a pregnancy status of a pregnant woman, and according to an embodiment of the present disclosure, with reference to FIG. 7, the apparatus includes: a parameter determination module 100 configured to determine predetermined parameters of the pregnant woman, the predetermined parameters including a concentration of fetal cell-free nucleic acids in peripheral blood of the pregnant woman and a gestational age in week at which sampling for the peripheral blood of the pregnant woman is conducted; and a pregnancy status determination module 200 connected to the parameter determination module 100 and configured to determine the pregnancy status of the pregnant woman based on the predetermined parameters and the prediction model. The apparatus according to the embodiment of the present disclosure can quickly and accurately predict the pregnancy status of the pregnant woman based on information about the concentration of fetal cell-free nucleic acids obtained via one-time blood sampling of the pregnant woman at early pregnancy, the gestational age in week at which the blood sampling is conducted, and the physical sign data of the pregnant woman, the pregnancy status including the gestational age in week at delivery, the probability of premature delivery, the intrauterine growth retardation of the fetus, and other pregnancy complications associated with the concentration of fetal cell-free nucleic acids. According to the apparatus of an embodiment of the present disclosure, the concentration of fetal cell-free nucleic acids is obtained by data processing using sequencing data of the cell-free nucleic acids in the plasma of the pregnant woman as input data, specifically including: after the quality control of raw sequencing data (fq format) is finished, aligning the sequencing data to human reference chromosomes by using alignment software (such as a samse mode in BWA);

using sequencing data quality control software (such as Picard) to remove the repeated reads in the alignment results and calculate the repetition rate; completing the local correction of the alignment results by using mutation detection algorithm (such as Base Quality Score Recalibration BQSR function in GATK); and calculating the average depth of different chromosomes in each sample by using coverage depth calculation software (such as Depth of Coverage function in GATK). For male fetus samples, the mean depth of coverage of the unique alignment reads matching the non-homologous region of Y chromosome is calculated, and the ratio of this mean depth to the mean depth of the unique alignment reads matching autosome is the concentration of fetal cell-free nucleic acids. For female fetus samples, calculation can be performed using existing methods for calculating the fetal concentration based on low-depth sequencing data of maternal plasma.

According to an embodiment of the present disclosure, the pregnancy status includes a delivery interval of the pregnant woman. The apparatus according to the embodiment of the present disclosure can be used to predict the probability of premature delivery, intrauterine growth retardation of a fetus at the gestational age in week at delivery, and other pregnancy complications associated with the concentration of fetal cell-free nucleic acids.

According to an embodiment of the present disclosure, the gestational age in week at which the sampling is conducted is 13 to 25 weeks. The inventors found that there was a weak correlation between fetal concentration and premature delivery when the gestational age in week at which the blood sampling is conducted is 12 weeks or less or between 26 weeks and 30 weeks, while there was a strong correlation between fetal concentration and premature delivery when the gestational age in week at which the blood sampling is conducted is 13 to 25 weeks. Generally, there is a problem of weak correlation in the prediction of the pregnancy status of pregnant women using the concentration of fetal cell-free nucleic acids. According to the apparatus of the embodiment of the present disclosure, the gestational age in week at which the sampling is conducted is added as one of the parameters for constructing the prediction model, which improves the accuracy of prediction, and blood sampling of the pregnant women only needs to be conducted once within the gestational age of 13 to 25 weeks, which reduces the cost and risk of multiple blood samplings.

According to an embodiment of the present disclosure, the predetermined prediction model is at least one of a linear regression model, a logistic regression model, or a random forest. According to the apparatus of an embodiment of the present disclosure, the prediction model may be theoretically any statistical model that generalizes different difference distributions.

According to a specific embodiment of the present disclosure, the predetermined parameters further include a height, a body weight, and an age of the pregnant woman, and the prediction model is adapted to calculate a delivery interval of the pregnant woman based on the following formula: $l = \beta_0 + \beta_{cff}x_{cff} + \beta_{sample}x_{sample} + \beta_{height}x_{height} + \beta_{weight}x_{weight} + \beta_{age}x_{age} + \varepsilon$, wherein $l$ is a parameter determined based on the probability of premature delivery of the pregnant woman; $\beta_0$, $\beta_{cff}$, $\beta_{sample}$, $\beta_{height}$, $\beta_{weight}$, and $\varepsilon$ are each independently a predetermined coefficient; $x_{cff}$ is the concentration of fetal cell-free nucleic acids of the pregnant woman; $x_{sample}$ is the gestational age in week at which the sampling for the peripheral blood of the pregnant woman is conducted; $x_{height}$ is the height of the pregnant woman; $x_{weight}$ is the body weight of the pregnant woman; $x_{age}$ is the age of the pregnant woman, and $\varepsilon$ is a sequencing error of a peripheral blood sample of the pregnant woman. According to an embodiment of the present disclosure, the coefficients $\beta_0$, $\beta_{cff}$, $\beta_{sample}$, $\beta_{height}$, and $\beta_{weight}$ may be freely selected as needed, for example, the pregnant woman BMI may be additionally added as one of the coefficients.

According to an embodiment of the present disclosure, $l$ is determined based on the following formula:

$$l = log_b \frac{P}{1-p}$$

, where b is a base number of log and is generally a constant e, and p is the probability of premature delivery of the pregnant woman.

In a fifth aspect of the present disclosure, provided is a computer-readable storage medium having a computer program stored thereon. The program, when executed by a processor, implements the steps of the above-described method for constructing the prediction model. Thus, the above-described method for constructing the prediction model can be effectively implemented, so that the prediction model can be effectively constructed, and the prediction model can be then used to perform prediction on an unknown sample to determine the pregnancy status of the pregnant woman to be detected.

In a sixth aspect of the present disclosure, provided is an electronic device including: the computer-readable storage medium; and one or more processors configured to execute the program in the computer-readable storage medium.

The present disclosure will be further explained below with reference to specific examples. The experimental methods applied in the following examples are conventional methods, unless otherwise specified. The materials, reagents, etc. used in the following examples are all commercially available, unless otherwise specified.

The technical solutions of the present disclosure will be explained below with reference to examples. Those skilled in the art will understand that these examples are illustrative only, and should not be considered as limiting the scope of the present disclosure. Examples, where specific techniques or conditions are not specified, are implemented in accordance with techniques or conditions described in the literature in the art (for example, refer to J. Sambrook et al. "Molecular Cloning: A Laboratory Manual" translated by Huang Peitang et al., 3rd edition, Science Press) or according to the product specification. All of the used reagents or instruments which are not specified with the manufacturer are conventional commercially-available products, for example, purchased from Illumina.

**Example 1 Construction and application of prediction model for premature delivery and gestational age in week at delivery**

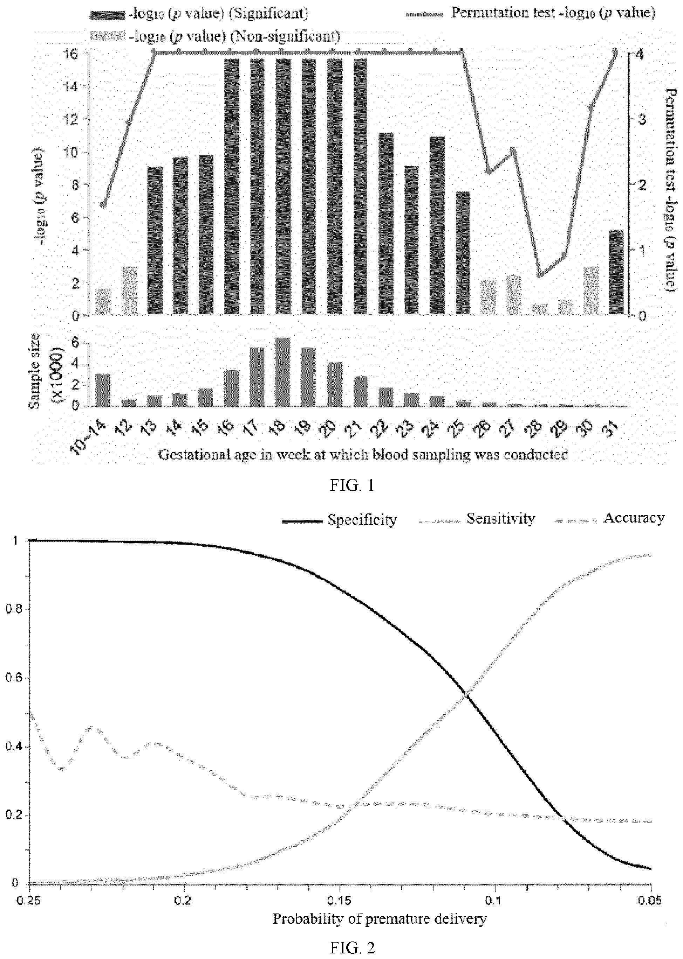

38964 samples were classified according to different gestational ages in week at which blood sampling was conducted, and the correlation between the concentration of fetal cfDNAs in plasma and the premature delivery was calculated respectively. With reference to FIG. 1, statistical analysis showed that the correlation between fetal concentration and premature delivery differed at different sampling gestational ages in week; there was a weak correlation between fetal concentration and premature delivery when the gestational age in week at which the blood sampling was conducted was 12 weeks or less or between 26 weeks and 30 weeks, while there was a strong correlation between fetal concentration and premature delivery when the gestational age in week at which the blood sampling was conducted was 13 to 25 weeks.

Plasma cfDNA data of 38964 pregnant women in combination with the gestational age in week at which the blood sampling was conducted and the age, height, and body weight information of the pregnant woman served as a training set:

(1) A linear regression model was established with the gestational age in week at delivery as a continuous variable in the prediction of the gestational age in week at delivery.

Specifically, by taking the gestational age in week at delivery as Y value, and taking the fetal cfDNA concentration, the gestational age in week at which the blood sampling was conducted, and the height, body weight, age and BMI of pregnant women as covariates, a prediction model was established: $y_i = \beta_0 + \beta_{icff}x_{icff} + \beta_{isample}x_{isample} + \beta_{iheight}x_{iheight} + \beta_{iweight}x_{iweight} + \beta_{iage}x_{iage} + \varepsilon_i$, *where i = 1, ..., p*, wherein $y_i$ is the gestational age in week at delivery corresponding to sample *i*, $x_{icff}$ is the fetal cfDNA concentration corresponding to sample *i*, $x_{isample}$ is the gestational age in week at which the blood sampling is conducted, corresponding to sample *i*, $x_{iheight}$ is the height of the pregnant woman corresponding to sample *i*, $x_{iweight}$ is the body weight of the pregnant woman corresponding to sample *i*, $x_{iage}$ is the age of the pregnant woman corresponding to sample *i*, $x_{ibmi}$ is the BMI of the pregnant woman corresponding to sample *i*, and *p* is the total number of samples in the training set, where *p* = 38964.

The estimated values of coefficient β for different variables in the finally obtained prediction model are shown in the column of gestational age in week at delivery in Table 2.

(2) A logistic regression model was established by defining premature delivery events as Y = 0 and defining full-term delivery events as Y = 1 in the prediction of premature delivery.

Specifically, the probability of full-term delivery of a sample was set as p = P (Y = 1), the probability of premature delivery of the sample was set as p = P (Y = 0), and this probability p was subjected to log-odds transformation, i.e.,

$$l = log_b \frac{P}{1-p}$$ , where b is the base number of log and is generally a constant e.

The transformed *l* was put into the linear regression model, and the fetal cfDNA concentration, gestational age in week at which blood sampling was conducted, and height, body weight, and age of pregnant women were also taken as covariates to establish a prediction model.

Specifically, by taking the gestational age in week at delivery as Y value, and taking the fetal cfDNA concentration, the gestational age in week at which blood sampling was conducted, and the height, body weight, age, and BMI of the pregnant women as covariates, a prediction model was established: $l_i = \beta_0 + \beta_{icff}x_{icff} + \beta_{isample}x_{isample} + \beta_{iheight}x_{iheight} + \beta_{iweight}x_{iweight} + \beta_{iage}x_{iage} + \varepsilon_i$, *where i = 1, ..., p,* wherein $l_i$ is the logical transformation result of the gestational age in week at delivery corresponding to sample *i*, $x_{icff}$ is the fetal cfDNA concentration corresponding to sample *i*, $x_{isample}$ is the gestational age in week at which blood sampling was conducted, corresponding to sample *i*, $x_{iheight}$ is the height of the pregnant woman corresponding to sample *i*, $x_{iweight}$ is the body weight of the pregnant woman corresponding to sample *i*, $x_{iage}$ is the age of the pregnant woman corresponding to sample *i*, $x_{ibmi}$ is the BMI of the pregnant woman corresponding to sample *i*, and p is the total number of samples in the training set, where *p* = 38964.

The estimated values of coefficient β for various variables in the finally obtained prediction model are shown in the column of premature delivery in Table 1.

Table 1. Statistical results of phenotype-related data of pregnant women in regression model for gestational age in week at delivery and regression model for premature delivery

| Predicted Value | Covariate | Estimated Value | Standard Deviation | Z/T Value | *p* value |
|---|---|---|---|---|---|
| Premature Delivery | Age of Pregnant Woman | -0.0461 | 0.0032 | -14.3160 | <2e-16 |
| | Height of Pregnant Woman | 0.0612 | 0.0225 | 2.7200 | 0.0065 |
| | Body Weight of Pregnant Woman | -0.0551 | 0.0299 | -1.8400 | 0.0657 |
| | BMI of Pregnant Woman | 0.1219 | 0.0774 | 1.5760 | 0.1151 |
| Gestational Age in Week at Delivery | Age of Pregnant Woman | -0.0407 | 0.0014 | -28.2810 | <2e-16 |
| | Height of Pregnant Woman | 0.0158 | 0.0100 | 1.5870 | 0.1120 |
| | Body Weight of Pregnant Woman | -0.0050 | 0.0134 | -0.3740 | 0.7080 |
| | BMI of Pregnant Woman | 0.0055 | 0.0349 | 0.1590 | 0.8740 |

After obtaining the prediction models for premature delivery and gestational age in week at delivery, additional 32049 samples were used as a test set, the fetal concentration, gestational age in week at which blood sampling was conducted, and age, height, body weight and BMI of pregnant woman corresponding to each sample were respectively put into the linear regression model to predict the gestational age in week at delivery and into the logistic regression model to predict premature delivery.

Refer to FIG. 2 for the accuracy of the finally obtained premature delivery prediction results, and refer to FIG. 3 for the distribution of the predicted gestational ages in week at delivery and the actual gestational ages in week at delivery. Wherein, the prediction results of premature delivery are significantly correlated with the actual results, with the correlation reaching -0.13, and the probability threshold for filtering can be determined according to the requirements of actual scenario for sensitivity and specificity. The correlation between the predicted gestational age in week at delivery and the actual gestational age in week at delivery reached 0.12.

In addition, reference to the term "an embodiment", "some embodiments", "an example", "a specific example" or "some examples" or the like means that a specific feature, structure, material, or characteristic described in combination with the example(s) or example(s) is included in at least one embodiment or example of the present disclosure. In this specification, illustrative expressions of these terms do not necessarily refer to the same embodiment or example. Moreover, the specific features, structures, materials, or characteristics described may be combined in any suitable manner in any one or more embodiments or examples. In addition, without mutual contradiction, those skilled in the art may combine different embodiments or examples and features of the different embodiments or examples described in this specification.

Although the embodiment or examples of the present disclosure have been illustrated and described above, it should be understood that the embodiments or examples are illustrative and should not be construed as limiting the present disclosure, and persons of ordinary skill in the art may make various changes, modifications, replacements and variations to the above embodiments or examples within the scope of the present disclosure.

Claims

1. A computer-implemented method for constructing a prediction model for determining a pregnancy status of a pregnant woman, the method comprising:

(i) constructing a training set and a selective validation set, each of the training set and the selective validation set being composed of a plurality of pregnant women samples each having a known pregnancy status;
(ii) determining predetermined parameters of each pregnant woman sample in the training set, the predetermined parameters comprising a concentration of fetal cell-free nucleic acids in peripheral blood of the pregnant woman sample and a gestational age in week at which sampling for the peripheral blood of the pregnant woman sample is conducted; and
(iii) constructing the prediction model based on the known pregnancy status and the predetermined parameters;

wherein the gestational age in week at which the sampling is conducted is 13 to 25 weeks.

2. The method according to claim 1, wherein the pregnancy status comprises a delivery interval of the pregnant woman.

3. The method according to claim 1, wherein the prediction model is at least one of a linear regression model, a logistic regression model, or a random forest.

4. The method according to claim 3, wherein the predetermined parameters further comprise a height, a body weight, and/or an age of the pregnant woman sample.

5. The method according to claim 1, wherein the step (iii) comprises:

determining, by using the training set and the selective validation set, numerical values of $\beta_0$, $\beta_{icff}$, $\beta_{isample}$, $\beta_{iheight}$, $\beta_{iweight}$, $\beta_{iage}$, and $\varepsilon_i$ for the following formula: $I_i = \beta_0 + \beta_{icff}x_{icff} + \beta_{isample}x_{isample} + \beta_{iheight}x_{iheight} + \beta_{iweight}x_{iweight} + \beta_{iage}x_{iage} + \varepsilon_i$, *where i = 1, ..., p*, wherein
*i* represents a serial number of the pregnant woman sample in the training set;
$I_i$ is a value determined for the known pregnancy status of the pregnant woman sample No.*i*, wherein $I_i$ is 1 for the pregnant woman sample with premature delivery, and $I_i$ is 0 for the pregnant woman sample with full-term delivery;
$x_{icff}$ represents the concentration of fetal cell-free nucleic acids for the pregnant woman sample *No.i;*
$x_{isample}$ represents the gestational age in week at which the sampling for the peripheral blood of the pregnant woman sample *No. i* is conducted;
$x_{iheight}$ represents a height of the pregnant woman sample *No. i;*
$x_{iweight}$ represents a body weight of the pregnant woman sample *No. i;*
$x_{iage}$ represents an age of the pregnant woman sample No.*i*; and
$\varepsilon_i$ represents a sequencing error of the peripheral blood of the pregnant woman sample *No. i.*

6. A system for constructing a prediction model for determining a pregnancy status of a pregnant woman, the system comprising:

a training set construction module configured to construct a training set composed of a plurality of pregnant woman samples each having a known pregnancy status;
a predetermined parameter determination module connected to the training set construction module and configured to determine predetermined parameters of each pregnant woman sample in the training set, the predetermined parameters comprising a concentration of fetal cell-free nucleic acids in peripheral blood of the pregnant woman and a gestational age in week at which sampling for the peripheral blood of the pregnant woman is conducted; and
a prediction model construction module connected to the predetermined parameter determination module and configured to construct the prediction model based on the known pregnancy status and the predetermined parameters.

7. The system according to claim 6, further satisfying any one of the following conditions:

the pregnancy status comprises a delivery interval of the pregnant woman;
the gestational age in week at which the sampling is conducted is 13 to 25 weeks; or
the prediction model is at least one of a linear regression model, a logistic regression model, or a random forest.

8. The system according to claim 6, wherein the prediction model is at least one of a linear regression model, a logistic regression model, or a random forest, and the predetermined parameters further a height, a body weight, and an age of the pregnant woman, preferably, the prediction model construction module is configured to determine, by using the training subset and a validation set, numerical values of $\beta_0$, $\beta_{icff}$, $\beta_{isample}$, $\beta_{iheight}$, $\beta_{iweight}$, $\beta_{iage}$, and $\varepsilon$ for the following formula:

$$I_i = \beta_0 + \beta_{icff}x_{icff} + \beta_{isample}x_{isample} + \beta_{iheight}x_{iheight} + \beta_{iweight}x_{iweight} + \beta_{iage}x_{iage} + \varepsilon_i, \text{ where } i = 1, ... , p,$$

wherein *i* represents a serial number of the pregnant woman sample in the training set;

$I_i$ is a value determined for the known pregnancy status of the pregnant woman sample No.*i*, wherein $I_i$ is 1 for the pregnant woman sample with premature delivery and $I_i$ is 0 for the pregnant woman sample with full-term delivery;
$x_{icff}$ represents the concentration of fetal cell-free nucleic acids for the pregnant woman sample No.*i*;
$x_{isample}$ represents the gestational age in week at which the sampling for the peripheral blood of the pregnant woman sample *No. i* is conducted;
$x_{iheight}$ represents a height of the pregnant woman sample *No. i;*

$x_{iweight}$ represents a body weight for the pregnant woman sample *No. i;*

$x_{iage}$ represents an age of the pregnant woman sample No.*i*; and

$\varepsilon_i$ represents a sequencing error of the peripheral blood of the pregnant woman sample *No. i.*

9. A method for determining a pregnancy status of a pregnant woman, comprising:

(1) determining predetermined parameters of the pregnant woman, the predetermined parameters comprising a concentration of fetal cell-free nucleic acids in peripheral blood of the pregnant woman and a gestational age in week at which sampling for the peripheral blood of the pregnant woman is conducted; and
(2) determining the pregnancy status of the pregnant woman based on the predetermined parameters and the prediction model constructed by the method according to any one of claims 1 to 5.

10. The method according to claim 9, wherein the pregnancy status comprises a delivery interval of the pregnant woman, the prediction model is at least one of a linear regression model, a logistic regression model, or a random forest, and the predetermined parameters further comprise a height, a body weight, and/or an age of the pregnant woman, and the prediction model is adapted to calculate the delivery interval of the pregnant woman based on the following formula:

$$l \ = \beta_0 + \beta_{cff}x_{cff} + \beta_{sample}x_{sample} + \beta_{height}x_{height} + \beta_{weight}x_{weight} + \beta_{age}x_{age} + \varepsilon,$$

wherein,

*l* is a parameter determined based on a probability of premature delivery of the pregnant woman;

$\beta_0$, $\beta_{cff}$, $\beta_{sample}$, $\beta_{height}$, $\beta_{weight}$, and $\varepsilon$ are each independently a predetermined coefficient;

$x_{cff}$ is the concentration of fetal cell-free nucleic acids of the pregnant woman;

$x_{sample}$ is the gestational age in week at which the sampling for the peripheral blood of the pregnant woman is conducted;

$x_{height}$ is the height of the pregnant woman;

$x_{weight}$ is the body weight of the pregnant woman;

$x_{age}$ is the age of the pregnant woman; and

$\varepsilon_i$ is a sequencing error of a peripheral blood sample of the pregnant woman,

wherein *l* is preferably determined based on the following formula:

$$l = log_b \frac{p}{1-p} \ ,$$

wherein,

b is a base number of log and is generally a constant e; and

p is the probability of premature delivery of the pregnant woman.

11. An apparatus for determining a pregnancy status of a pregnant woman, comprising:

a parameter determination module configured to determine predetermined parameters of the pregnant woman, the predetermined parameters comprising a concentration of fetal cell-free nucleic acids in peripheral blood of the pregnant woman and a gestational age in week at which sampling for the peripheral blood of the pregnant woman is conducted; and
a pregnancy status determination module connected to the parameter determination module and configured to determine the pregnancy status of the pregnant woman based on the predetermined parameters and the system according to any one of claims 6 to 8.

12. The apparatus according to claim 12, wherein the predetermined parameters further comprise a height, a body weight, and/or an age of the pregnant woman, and the prediction model is adapted to calculate a delivery interval of the pregnant woman based on the following formula:

$$l \ = \beta_0 + \beta_{cff}x_{cff} + \beta_{sample}x_{sample} + \beta_{height}x_{height} + \beta_{weight}x_{weight} + \beta_{age}x_{age} + \varepsilon,$$

wherein,

*l* is a parameter determined based on a probability of premature delivery of the pregnant woman;

$\beta_0$, $\beta_{cff}$, $\beta_{sample}$, $\beta_{height}$, $\beta_{weight}$ and $\varepsilon$ are each independently a predetermined coefficient;

$x_{cff}$ is the concentration of fetal cell-free nucleic acids of the pregnant woman;

$x_{sample}$ is the gestational age in week at which the sampling for the maternal peripheral blood of the pregnant woman is conducted;

$x_{height}$ is the height of the pregnant woman;

$x_{weight}$ is the body weight of the pregnant woman;

$x_{age}$ is the age of the pregnant woman; and

$\varepsilon$ is a sequencing error of a peripheral blood sample of the pregnant woman,

wherein *l* is preferably determined based on the following formula:

$$l = log_b \frac{P}{1-p} \quad ,$$

wherein,

b is a base number of log and is generally a constant e; and

p is the probability of premature delivery of the pregnant woman.

13. A computer-readable storage medium, having a computer program stored thereon, wherein the program, when executed by a processor, implements steps of the method according to any one of claims 1 to 5 or the method according to claim 9 or 10.

**Patentansprüche**

1. Computerimplementiertes Verfahren zum Erstellen eines Vorhersagemodells zur Bestimmung eines Schwangerschaftsstatus einer schwangeren Frau, umfassend Folgendes:

(i) Erstellen eines Trainingssatzes und eines selektiven Validierungssatzes, wobei jeder von dem Trainingssatz und dem selektiven Validierungssatz aus mehreren Proben von schwangeren Frauen besteht, die jeweils einen bekannten Schwangerschaftsstatus aufweisen;

(ii) Bestimmen vorgegebener Parameter für jede der Proben von schwangeren Frauen im Trainingssatz, wobei die vorgegebenen Parameter eine Konzentration von fetalen zellfreien Nukleinsäuren im peripheren Blut der Probe der schwangeren Frau und eine Schwangerschaftswoche, in der die Probeentnahme für das periphere Blut der Probe der schwangeren Frau durchgeführt wird, umfassen; und

(iii) Erstellen des Vorhersagemodells basierend auf dem bekannten Schwangerschaftsstatus und den vorgegebenen Parametern;

wobei die Schwangerschaftswoche, in der die Probeentnahme durchgeführt wird, zwischen der 13 und 25 Wochen liegt.

2. Verfahren nach Anspruch 1, wobei der Schwangerschaftsstatus ein Entbindungsintervall der schwangeren Frau umfasst.

3. Verfahren nach Anspruch 1, wobei das Vorhersagemodell mindestens eines von einem linearen Regressionsmodell, einem logistischen Regressionsmodell oder einem Random Forest ist.

4. Verfahren nach Anspruch 3, wobei die vorgegebenen Parameter ferner eine Körpergröße, ein Körpergewicht und/oder ein Alter der Probe der schwangeren Frau umfassen.

5. Verfahren nach Anspruch 1, wobei der Schritt (iii) Folgendes umfasst:

Bestimmen der numerischen Werte von $\beta_0$, $\beta_{icff}$, $\beta_{isample}$, $\beta_{iheight}$, $\beta_{iweight}$, $\beta_{iage}$ und $\varepsilon_i$ für die folgende Formel: $l_i = \beta_0 + \beta_{icff}x_{icff} + \beta_{isample}x_{isample} + \beta_{iheight}x_{iheight} + \beta_{iweight}x_{iweight} + \beta_{iage}x_{iage} + \varepsilon_i$, where $i = 1, ... , p$ unter Verwendung des Trainingssatzes und des selektiven Validierungssatzes, wobei

*i* eine Seriennummer der Probe der schwangeren Frau im Trainingssatz repräsentiert;

$l_i$ ein Wert ist, der für den bekannten Schwangerschaftsstatus der Probe der schwangeren Frau Nr. *i* bestimmt wird, wobei $l_i$ für die Probe der schwangeren Frau mit Frühgeburt 1 ist und $l_i$ für die Probe der schwangeren Frau mit Termingeburt 0 ist;

$x_{icff}$ die Konzentration von fetalen zellfreien Nukleinsäuren für die Probe der schwangeren Frau Nr. *i* repräsentiert;

$x_{isample}$ die Schwangerschaftswoche repräsentiert, in der die Probeentnahme für das periphere Blut der Probe der schwangeren Frau Nr. *i* durchgeführt wird;

$x_{iheight}$ eine Körpergröße der Probe der schwangeren Frau Nr. *i* repräsentiert;

$x_{iweight}$ ein Körpergewicht der Probe der schwangeren Frau Nr. *i* repräsentiert;

$x_{iage}$ ein Alter der Probe der schwangeren Frau Nr. *i* repräsentiert; und

$\varepsilon_i$ einen Sequenzierungsfehler des peripheren Blutes der Probe der schwangeren Frau Nr. *i* repräsentiert.

6. System zum Erstellen eines Vorhersagemodells zur Bestimmung eines Schwangerschaftsstatus einer schwangeren Frau, umfassend:

ein Trainingssatz-Erstellungsmodul, das zum Erstellen eines Trainingssatzes konfiguriert ist, der aus mehreren Proben von schwangeren Frauen mit jeweils einem bekannten Schwangerschaftsstatus besteht;

ein Modul zur Bestimmung von vorgegebenen Parametern, das mit dem Trainingssatz-Erstellungsmodul verbunden ist und dazu konfiguriert ist, vorgegebene Parameter für jede der Proben von schwangeren Frauen im Trainingssatz zu bestimmen, wobei die vorgegebenen Parameter eine Konzentration von fetalen zellfreien Nukleinsäuren im peripheren Blut der schwangeren Frau und eine Schwangerschaftswoche, in der die Probeentnahme für das periphere Blut der schwangeren Frau durchgeführt wird, umfassen; und

ein Modul zur Erstellung eines Vorhersagemodells, das mit dem Modul zur Bestimmung von vorgegebenen Parametern verbunden ist und dazu konfiguriert ist, das Vorhersagemodell basierend auf dem bekannten Schwangerschaftsstatus und den vorgegebenen Parametern zu erstellen.

7. System nach Anspruch 6, das ferner eine der folgenden Bedingungen erfüllt:

der Schwangerschaftsstatus einen Entbindungsintervall der schwangeren Frau umfasst;

die Schwangerschaftswoche, in der die Probeentnahme durchgeführt wird, zwischen der 13 und Wochen liegt; oder

das Vorhersagemodell mindestens eines von einem linearen Regressionsmodell, einem logistischen Regressionsmodell oder einem Random Forest ist.

8. System nach Anspruch 6, wobei das Vorhersagemodell mindestens eines von einem linearen Regressionsmodell, einem logistischen Regressionsmodell oder einem Random Forest ist und die vorgegebenen Parameter ferner eine Körpergröße, ein Körpergewicht und/oder ein Alter der schwangeren Frau umfassen, wobei das Modell zur Erstellung des Vorhersagemodells vorzugsweise dazu konfiguriert ist, unter Verwendung des Trainings-Teilsatzes und eines Validierungssatzes numerische Werte von $\beta_0$, $\beta_{icff}$, $\beta_{isample}$, $\beta_{iheight}$, $\beta_{iweight}$, $\beta_{iage}$ und $\varepsilon_i$ für die folgende Formel: $l_i = \beta_0 + \beta_{icff}x_{icff} + \beta_{isample}x_{isample} + \beta_{iheight}x_{iheight} + \beta_{iweight}x_{iweight} + \beta_{iage}x_{iage} + \varepsilon_i$, *where i* = 1, ... , *p* zu bestimmen, wobei *i* eine Seriennummer der Probe der schwangeren Frau im Trainingssatz repräsentiert;

$l_i$ ein Wert ist, der für den bekannten Schwangerschaftsstatus der Probe der schwangeren Frau Nr. *i* bestimmt wird, wobei $l_i$ für die Probe der schwangeren Frau mit Frühgeburt 1 ist und $l_i$ für die Probe der schwangeren Frau mit Termingeburt 0 ist;

$x_{icff}$ die Konzentration von fetalen zellfreien Nukleinsäuren für die Probe der schwangeren Frau Nr. *i* repräsentiert;

$x_{isample}$ die Schwangerschaftswoche repräsentiert, in der die Probeentnahme für das periphere Blut der Probe der schwangeren Frau Nr. *i* durchgeführt wird;

$x_{iheight}$ eine Körpergröße der Probe der schwangeren Frau Nr. *i* repräsentiert;

$x_{iweight}$ ein Körpergewicht der Probe der schwangeren Frau Nr. *i* repräsentiert;

$x_{iage}$ ein Alter der Probe der schwangeren Frau Nr. *i* repräsentiert; und

$\varepsilon_i$ einen Sequenzierungsfehler des peripheren Blutes der Probe der schwangeren Frau Nr. *i* repräsentiert.

9. Verfahren zur Bestimmung eines Schwangerschaftsstatus einer schwangeren Frau, das Folgendes umfasst:

(1) Bestimmen vorgegebener Parameter der schwangeren Frau, wobei die vorgegebenen Parameter eine Konzentration von fetalen zellfreien Nukleinsäuren im peripheren Blut der schwangeren Frau und eine Schwangerschaftswoche, in der die Probeentnahme für das periphere Blut der schwangeren Frau durchgeführt wird, umfassen; und

(2) Bestimmen des Schwangerschaftsstatus der schwangeren Frau basierend auf den vorgegebenen Parametern und dem Vorhersagemodell, das durch das Verfahren gemäß einem der Ansprüche 1 bis 5 erstellt wird.

10. Verfahren nach Anspruch 9, wobei der Schwangerschaftsstatus ein Entbindungsintervall der schwangeren Frau umfasst, das Vorhersagemodell mindestens eines von einem linearen Regressionsmodell, einem logistischen Regressionsmodell oder einem Random Forest ist und die vorgegebenen Parameter ferner eine Körpergröße, ein Körpergewicht und/oder ein Alter der schwangeren Frau umfassen, und wobei das Vorhersagemodell geeignet ist, das Entbindungsintervall der schwangeren Frau anhand der folgenden Formel zu berechnen:

$$l_{\square} = \beta_0 + \beta_{cff}x_{cff} + \beta_{sample}x_{sample} + \beta_{height}x_{height} + \beta_{weight}x_{weight} + \beta_{age}x_{age} + \varepsilon,$$

wobei,

*l* ein Parameter ist, der basierend auf einer Wahrscheinlichkeit für eine Frühgeburt der schwangeren Frau bestimmt wird;
$\beta_0$, $\beta_{cff}$, $\beta_{sample}$, $\beta_{height}$, $\beta_{weight}$ und $\varepsilon$ jeweils unabhängig voneinander einen vorgegebenen Koeffizienten sind;
$x_{cff}$ die Konzentration von fetalen zellfreien Nukleinsäuren der schwangeren Frau ist; $x_{sample}$ die Schwangerschaftswoche ist, in der die Probeentnahme für das periphere Blut der schwangeren Frau durchgeführt wird;
$x_{height}$ die Körpergröße der schwangeren Frau ist;
$x_{weight}$ die Körpergewicht der schwangeren Frau ist;
$x_{age}$ das Alter der schwangeren Frau ist; und
$\varepsilon_i$ einen Sequenzierungsfehler einer peripheren Blutprobe der schwangeren Frau ist,
wobei *l* vorzugsweise anhand der folgenden Formel bestimmt wird:

$$l = log_b \frac{p}{1-p},$$

wobei,

b eine Basenzahl von log und im Allgemeinen eine Konstante e ist; und
p die Wahrscheinlichkeit für eine Frühgeburt der schwangeren Frau ist.

11. Vorrichtung zur Bestimmung eines Schwangerschaftsstatus einer schwangeren Frau, umfassend:

ein Modul zur Parameterbestimmung, das dazu konfiguriert ist, vorgegebene Parameter der schwangeren Frau zu bestimmen, wobei die vorgegebenen Parameter eine Konzentration von fetalen zellfreien Nukleinsäuren im peripheren Blut der schwangeren Frau und eine Schwangerschaftswoche, in der die Probeentnahme für das periphere Blut der schwangeren Frau durchgeführt wird, umfassen; und
ein Modul zur Bestimmung des Schwangerschaftsstatus, das mit dem Modul zur Parameterbestimmung verbunden ist und dazu konfiguriert ist, den Schwangerschaftsstatus der schwangeren Frau basierend auf den vorgegebenen Parametern und dem System gemäß einem der Ansprüche 6 bis 8 zu bestimmen.

12. Vorrichtung nach Anspruch 12, wobei die vorgegebenen Parameter ferner eine Körpergröße, ein Körpergewicht und/oder ein Alter der schwangeren Frau umfassen, und wobei das Vorhersagemodell geeignet ist, das Entbindungsintervall der schwangeren Frau anhand der folgenden Formel zu berechnen:

$$l_{\square} = \beta_0 + \beta_{cff}x_{cff} + \beta_{sample}x_{sample} + \beta_{height}x_{height} + \beta_{weight}x_{weight} + \beta_{age}x_{age} + \varepsilon,$$

wobei,

*l* ein Parameter ist, der basierend auf einer Wahrscheinlichkeit für eine Frühgeburt der schwangeren Frau bestimmt wird;
$\beta_0$, $\beta_{cff}$, $\beta_{sample}$, $\beta_{height}$, $\beta_{weight}$ und $\varepsilon$ jeweils unabhängig voneinander einen vorgegebenen Koeffizienten sind;
$x_{cff}$ die Konzentration von fetalen zellfreien Nukleinsäuren der schwangeren Frau ist; $x_{sample}$ die Schwangerschaftswoche ist, in der die Probeentnahme für das mütterliche periphere Blut der schwangeren Frau durchgeführt wird;
$x_{height}$ die Körpergröße der schwangeren Frau ist;

$x_{weight}$ die Körpergewicht der schwangeren Frau ist;

$x_{age}$ das Alter der schwangeren Frau ist; und

$\varepsilon_i$ einen Sequenzierungsfehler einer peripheren Blutprobe der schwangeren Frau ist,

wobei *l* vorzugsweise anhand der folgenden Formel bestimmt wird:

$$l = log_b \frac{p}{1-p},$$

wobei,

b eine Basenzahl von log und im Allgemeinen eine Konstante e ist; und

p die Wahrscheinlichkeit für eine Frühgeburt der schwangeren Frau ist

13. Computerlesbares Speichermedium, auf dem ein Computerprogramm gespeichert ist, wobei das Programm beim Durchführen durch einen Prozessor Schritte des Verfahrens gemäß einem der Ansprüche 1 bis 5 oder des Verfahrens gemäß Anspruch 9 oder 10 implementiert.

**Revendications**

1. Un procédé mis en œuvre par ordinateur de construction d'un modèle de prédiction pour déterminer le statut de grossesse d'une femme enceinte, le procédé comprenant :

   (i) la construction d'un ensemble d'entraînement et d'un ensemble de validation sélective, chacun des ensembles d'entraînement et de validation sélective étant composé d'une pluralité d'échantillons de femmes enceintes ayant chacun un statut de grossesse connu ;
   (ii) la détermination de paramètres prédéterminés de chaque échantillon de femme enceinte dans l'ensemble d'entraînement, les paramètres prédéterminés comprenant une concentration d'acides nucléiques fœtaux acellulaires dans le sang périphérique de l'échantillon de femme enceinte et un âge gestationnel en semaines auquel le prélèvement de sang périphérique de l'échantillon de femme enceinte est effectué ; et
   (iii) la construction du modèle de prédiction basé sur le statut de grossesse connu et les paramètres prédéterminés ;

   dans lequel l'âge gestationnel en semaines auquel le prélèvement est effectué est de 13 à 25 semaines.

2. Le procédé selon la revendication 1, **caractérisé en ce que** le statut de grossesse comprend un intervalle d'accouchement de la femme enceinte.

3. Le procédé selon la revendication 1, **caractérisé en ce que** le modèle de prédiction est au moins l'un des suivants : un modèle de régression linéaire, un modèle de régression logistique ou une forêt aléatoire.

4. Le procédé selon la revendication 3, **caractérisé en ce que** les paramètres prédéterminés comprennent en outre une taille, un poids corporel et/ou un âge de l'échantillon de femme enceinte.

5. Le procédé selon la revendication 1, **caractérisé en ce que** l'étape (iii) comprend :

   déterminer, à l'aide de l'ensemble d'entraînement et de l'ensemble de validation sélective, les valeurs numériques de $\beta_0$, et $\beta_{icff}$, $\beta_{isample}$, $\beta_{iheight}$, $\beta_{iweight}$, $\beta_{iage}$, et $\varepsilon_i$ coefficients pour la formule suivante : $l_i = \beta_0 + \beta_{icff}x_{icff} + \beta_{isample}x_{isample} + \beta_{iheight}x_{iheight} + \beta_{iweight}x_{iweight} + \beta_{iage}x_{iage} + \varepsilon_i$, où $i = 1, ..., p$, dans laquelle
   *i* représente un numéro de série de l'échantillon de femme enceinte dans l'ensemble d'entraînement ;
   $l_i$ est une valeur déterminée pour le statut de grossesse connu de l'échantillon de femme enceinte n°*i*, dans lequel $l_i$ est de 1 pour l'échantillon de femme enceinte ayant accouché prématurément, et $l_i$ est de 0 pour l'échantillon de femme enceinte ayant accouché à terme ;
   $x_{icff}$ représente la concentration d'acides nucléiques acellulaires fœtaux pour l'échantillon de femme enceinte n°*i* ;
   $x_{isample}$ représente l'âge gestationnel en semaines auquel le prélèvement de sang périphérique de l'échantillon de femme enceinte n°*i* est effectué ;
   $x_{iheight}$ représente la taille de l'échantillon de femme enceinte n°*i* ;
   $x_{iweight}$ représente le poids corporel de l'échantillon de femme enceinte n°*i* ;

$x_{iage}$ représente l'âge de l'échantillon de femme enceinte n°$i$ ; et

$\varepsilon_i$ représente une erreur de séquençage du sang périphérique de l'échantillon n°$i$ de la femme enceinte.

6. Un système pour construire un modèle de prédiction pour déterminer le statut de grossesse d'une femme enceinte, le système comprenant :

un module de construction d'ensemble d'entraînement configuré pour construire un ensemble d'entraînement composé d'une pluralité d'échantillons de femmes enceintes, chacun ayant un statut de grossesse connu ;
un module de détermination de paramètres prédéterminés connecté au module de construction de l'ensemble d'entraînement et configuré pour déterminer des paramètres prédéterminés de chaque échantillon de femme enceinte dans l'ensemble d'entraînement, les paramètres prédéterminés comprenant une concentration d'acides nucléiques fœtaux acellulaires dans le sang périphérique de la femme enceinte et un âge gestationnel en semaines auquel le prélèvement de sang périphérique de la femme enceinte est effectué ; et
un module de construction de modèle de prédiction connecté au module de détermination de paramètres prédéterminés et configuré pour construire le modèle de prédiction basé sur le statut de grossesse connu et les paramètres prédéterminés.

7. Le système selon la revendication 6, **caractérisé en ce qu'**il satisfait en outre l'une quelconque des conditions suivantes :

le statut de grossesse comprend un intervalle d'accouchement de la femme enceinte ;
l'âge gestationnel en semaines auquel le prélèvement est effectué est de 13 à 25 semaines ; ou
le modèle de prédiction est au moins l'un d'un modèle de régression linéaire, d'un modèle de régression logistique ou d'une forêt aléatoire.

8. Le système selon la revendication 6, **caractérisé en ce que** le modèle de prédiction est au moins l'un d'un modèle de régression linéaire, d'un modèle de régression logistique ou d'une forêt aléatoire, et les paramètres prédéterminés comprennent en outre une taille, un poids corporel et un âge de la femme enceinte ; de préférence, le module de construction de modèle de prédiction est configuré pour déterminer, à l'aide d'un sous-ensemble d'entraînement et d'un ensemble de validation, les valeurs numériques de $\beta_0$, et $\beta_{icff}$, $\beta_{isample}$, $\beta_{iheight}$, $\beta_{iweight}$, $\beta_{iage}$, et $\varepsilon$ coefficients pour la formule suivante :

$$l_i = \beta_0 + \beta_{icff}x_{icff} + \beta_{isample}x_{isample} + \beta_{iheight}x_{iheight} + \beta_{iweight}x_{iweight} + \beta_{iage}x_{iage} + \varepsilon_i,\ \text{où } i = 1, \dots, p,$$

dans laquelle

$i$ représente un numéro de série de l'échantillon de femme enceinte dans l'ensemble d'entraînement ;
$l_i$ est une valeur déterminée pour le statut de grossesse connu de l'échantillon de femme enceinte n°$i$, dans lequel $l_i$ est de 1 pour l'échantillon de femme enceinte ayant accouché prématurément et $l_i$ est de 0 pour l'échantillon de femme enceinte ayant accouché à terme ;
$x_{icff}$ représente la concentration d'acides nucléiques acellulaires fœtaux pour l'échantillon de femme enceinte n°$i$ ;
$x_{isample}$ représente l'âge gestationnel en semaines auquel le prélèvement de sang périphérique de l'échantillon de femme enceinte n°$i$ est effectué ;
$x_{iheight}$ représente la taille de l'échantillon de femme enceinte n°$i$ ;
$x_{iweight}$ représente le poids corporel de l'échantillon de femme enceinte n°$i$ ;
$x_{iage}$ représente l'âge de l'échantillon de femme enceinte n°$i$ ; et
$\varepsilon_i$ représente une erreur de séquençage du sang périphérique de l'échantillon n°$i$ de la femme enceinte.

9. Une méthode pour déterminer le statut de grossesse d'une femme enceinte, comprenant :

(1) la détermination de paramètres prédéterminés de la femme enceinte, les paramètres prédéterminés comprenant une concentration d'acides nucléiques fœtaux acellulaires dans le sang périphérique de la femme enceinte et un âge gestationnel en semaines auquel le prélèvement de sang périphérique de la femme enceinte est effectué ; et
(2) la détermination du statut de grossesse de la femme enceinte basé sur les paramètres prédéterminés et le modèle de prédiction construit par la méthode selon l'une quelconque des revendications 1 à 5.

**10.** La méthode selon la revendication 9, **caractérisée en ce que** le statut de grossesse comprend un intervalle d'accouchement de la femme enceinte, le modèle de prédiction est au moins l'un des suivants : un modèle de régression linéaire, un modèle de régression logistique ou une forêt aléatoire, et les paramètres prédéterminés comprennent en outre une taille, un poids corporel et/ou un âge de la femme enceinte ; le modèle de prédiction est adapté pour calculer l'intervalle d'accouchement de la femme enceinte basé sur la formule suivante :

$$l_{\square} = \beta_0 + \beta_{cff}x_{cff} + \beta_{sample}x_{sample} + \beta_{height}x_{height} + \beta_{weight}x_{weight} + \beta_{age}x_{age} + \varepsilon,$$

dans laquelle

*l* est un paramètre déterminé en fonction d'une probabilité d'accouchement prématuré de la femme enceinte ;
$\beta_0$, $\beta_{cff}$, $\beta_{sample}$, $\beta_{height}$, $\beta_{weight}$, et $\varepsilon$ sont chacun indépendamment un coefficient prédéterminé ;
$x_{cff}$ est la concentration d'acides nucléiques fœtaux acellulaires de la femme enceinte ;
$x_{sample}$ est l'âge gestationnel en semaines auquel le prélèvement de sang périphérique de la femme enceinte est effectué ;
$x_{height}$ est la taille de la femme enceinte ;
$x_{weight}$ est le poids corporel de la femme enceinte ;
$x_{age}$ est l'âge de la femme enceinte ; et
$\varepsilon_i$ est une erreur de séquençage d'un échantillon de sang périphérique de la femme enceinte ; dans lequel *l* est de préférence déterminé selon la formule suivante :

$$l = log_b \frac{p}{1-p},$$

dans laquelle
b est une base du logarithme et est généralement la constante e ; et
p est la probabilité d'accouchement prématuré de la femme enceinte.

**11.** Un appareil pour déterminer le statut de grossesse d'une femme enceinte, comprenant :

un module de détermination de paramètres configuré pour déterminer des paramètres prédéterminés de la femme enceinte, les paramètres prédéterminés comprenant une concentration d'acides nucléiques fœtaux acellulaires dans le sang périphérique de la femme enceinte et un âge gestationnel en semaines auquel le prélèvement de sang périphérique de la femme enceinte est effectué ; et
un module de détermination du statut de grossesse connecté au module de détermination de paramètres et configuré pour déterminer le statut de grossesse de la femme enceinte basé sur les paramètres prédéterminés et le système selon l'une quelconque des revendications 6 à 8.

**12.** L'appareil selon la revendication 12, **caractérisé en ce que** les paramètres prédéterminés comprennent en outre une taille, un poids corporel et/ou un âge de la femme enceinte, et le modèle de prédiction est adapté pour calculer un intervalle d'accouchement de la femme enceinte basé sur la formule suivante :

$$l_{\square} = \beta_0 + \beta_{cff}x_{cff} + \beta_{sample}x_{sample} + \beta_{height}x_{height} + \beta_{weight}x_{weight} + \beta_{age}x_{age} + \varepsilon,$$

dans laquelle

*l* est un paramètre déterminé en fonction d'une probabilité d'accouchement prématuré de la femme enceinte ;
$\beta_0$, $\beta_{cff}$, $\beta_{sample}$, $\beta_{height}$, $\beta_{weight}$ et $\varepsilon$ sont chacun indépendamment un coefficient prédéterminé ;
$x_{cff}$ est la concentration d'acides nucléiques fœtaux acellulaires de la femme enceinte ;
$x_{sample}$ est l'âge gestationnel en semaines auquel le prélèvement de sang périphérique maternel de la femme enceinte est effectué ;
$x_{height}$ est la taille de la femme enceinte ;
$x_{weight}$ est le poids corporel de la femme enceinte ;
$x_{age}$ est l'âge de la femme enceinte ; et
$\varepsilon$ est une erreur de séquençage d'un échantillon de sang périphérique de la femme enceinte ;
dans leque *l* est de préférence déterminé selon la formule suivante :*l*

$$l = log_b \frac{P}{1-p},$$

dans laquelle

b est une base du logarithme et est généralement la constante e ; et

p est la probabilité d'accouchement prématuré de la femme enceinte.

13. Un support de stockage lisible par ordinateur, **caractérisé en ce qu'**il stocke un programme informatique, lequel programme, lorsqu'il est exécuté par un processeur, met en œuvre les étapes du procédé selon l'une quelconque des revendications 1 à 5 ou du procédé selon la revendication 9 ou 10.

FIG. 1

FIG. 2

Actual gestational age in week at delivery

FIG. 3

FIG. 4

Training set
construction module

1000

Predetermined
parameter
determination module

2000

Prediction model
construction module

3000

FIG. 5

Determining
predetermined parameters
of a pregnant woman

S100

Determining the
pregnancy status of the
pregnant woman

S200

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7541182 B2

**Non-patent literature cited in the description**

- **LIN JASON et al.** *Predicting Premature Birth Risk with cf RNA Recommended Citation Predicting Premature Birth Risk with cfRNA*

- **J. SAMBROOK et al.** Molecular Cloning: A Laboratory Manual. Science Press